Europäisches Patentamt
European Patent Office
Office européen des brevets

(19)

(11) Veröffentlichungsnummer: 0 516 978 A1

(12) **EUROPÄISCHE PATENTANMELDUNG**

(21) Anmeldenummer: **92107394.6**

(22) Anmeldetag: **30.04.92**

(51) Int. Cl.5: **C07D 333/20**, C07B 57/00, A61K 31/38

(30) Priorität: **03.05.91 DE 4114444**

(43) Veröffentlichungstag der Anmeldung: **09.12.92 Patentblatt 92/50**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Elben, Ulrich, Dr.**
**Stettiner Strasse 15/4**
**W-6203 Hochheim am Main(DE)**
Erfinder: **Anagnostopulos, Hiristo, Dr.**
**Forststrasse 40a**
**W-6200 Wiesbaden(DE)**
Erfinder: **Rudolphi, Karl, Dr.**
**Kapitän-Strasser-Strasse 27**
**W-6078 Neu-Isenburg(DE)**

(54) **Enantiomere von substituierten (Aminophenyl)--hetroarylethylaminen, Verfahren zu deren Herstellung und diese Verbindungen enthaltende Arzneimittel.**

(57) Die Erfindung betrifft (-)-Enantiomere der Verbindung der Formel I,

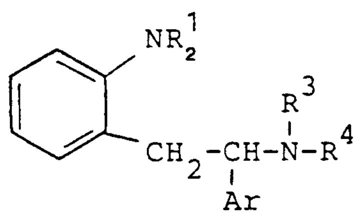

(I)

Verfahren zur Herstellung der (-)-Enantiomere, sowie deren Verwendung als Arzneimittel zur Prophylaxe und/oder Therapie von neurodegenerativen Erkrankungen.

EP 0 516 978 A1

Die Erfindung betrifft (-)-Enantiomere der Verbindung der Formel I

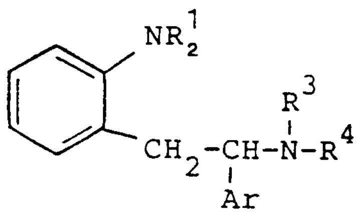

(I)

die spezifische Antagonisten des N-Methyl-D-aspartat (NMDA)-Rezeptors sind und sich daher für die Behandlung von Krankheitsformen als Konsequenz von Erkrankungen wie Hirninfarkt, Hypoglykämie, zerebralen Tremor, temporärer Hirnischämie (TIA), zerebralem Infarkt mit nur teilweise reversiblen Symptomen (PRIND), ischämischen Zuständen nach Herzstillstand oder chirurgischen Eingriffen, anoxischen Zuständen, Intoxikationen oder Rückenmarksverletzungen, perinataler Asphyxie, altersbedingter neurodegenerativer Erkrankungen, Alzheimer Demenz (SDAT), Schizophrenie, Epilepsie, Huntington-Chorea, Amylotrophische Lateralsklerose, Parkinsonsche Erkrankung sowie anderer neurodegenerativer Erkrankungen eignen.

Aus der Europäischen Offenlegungsschrift 322 582 ist bekannt, daß Verbindungen der Formel I antikonvulsive Wirkungen haben.Das die Ar-Gruppe tragende C-Atom in der Seitenkette ist asymmetrisch. Die in dieser Offenlegungsschritt untersuchten Verbindungen liegen jedoch ausschließlich in Form ihrer Razemate vor. Über entsprechende Enantiomere wurde aber noch nicht berichtet. Es wurde nun eine Methode gefunden, die optisch aktiven Verbindungen dieser Razemate herzustellen. Überraschenderweise wurde gefunden, daß das (-)-Enantiomer der Verbindung der Formel I eine stärkere Schutzwirkung als das entsprechende Razemat, sowie eine wesentlich geringere Beeinträchtigung des zentralen Nervensystems (ZNS) aufweist; daher höher dosiert werden kann und dadurch zu einem besseren neuroprotektivem Schutz führt als das Razemat oder das (+)-Enantiomer.

Die Erfindung betrifft somit (-)-Enantiomere der Verbindung der Formel I,

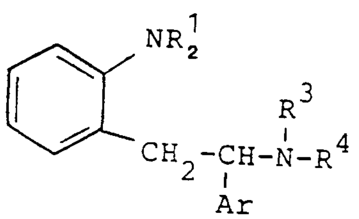

(I)

wobei Ar für Verbindungen aus der Gruppe

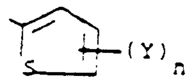 , 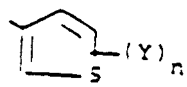 , 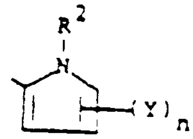 , 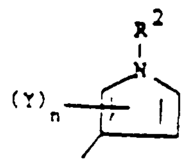

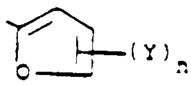 , 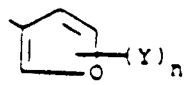 ,

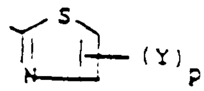 , 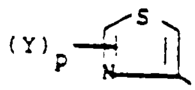 , 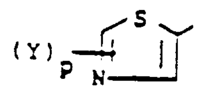 ,

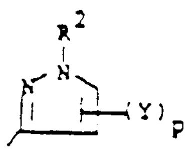 , 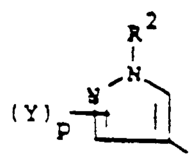 , 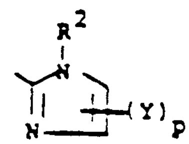 ,

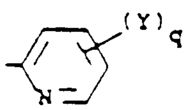 , 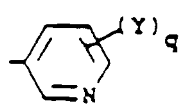 , 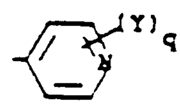

worin Y für
- 1) Halogen, wie Fluor, Chlor, Brom oder Jod,
- 2) Hydroxy,
- 3) ($C_1$-$C_5$)-Alkyl, geradkettig oder verzweigt,
- 4) ($C_1$-$C_5$)-Alkoxy, geradkettig oder verzweigt,
- 5) Trifluormethyl,

n für eine ganze Zahl von 0 bis 3,
p für eine ganze Zahl von 0 oder 1,
q für eine ganze Zahl von 0 bis 4,
$R^2$ für
- 1) Wasserstoff,
- 2) ($C_1$-$C_5$)-Alkyl, geradkettig oder verzweigt,
- 3) ($C_1$-$C_5$)-Alkanoyl, geradkettig oder verzweigt,

$R^1$ für
- 1) Wasserstoff,
- 2) ($C_1$-$C_5$)-Alkyl,

$R^3$ für
- 1) Wasserstoff,
- 2) ($C_1$-$C_5$)-Alkyl,
- 3) Phenyl,
- 4) Phenyl, ein- oder mehrfach substituiert durch
  - 4.1 ($C_1$-$C_4$)-Alkyl
  - 4.2 Halogen, wie Fluor, Chlor oder Brom,
  - 4.3 Methoxy,
  - 4.4 Hydroxy,
  - 4.5 Trifluormethyl,

$R^4$ für
- 1) Wasserstoff,
- 2) ($C_1$-$C_5$)-Alkyl,

3) -C(O)-$R^5$,

$R^5$ für

1) Wasserstoff,

2) ($C_1$-$C_5$)-Alkyl,

3) Allyl,

steht und/oder deren pharmazeutisch verträgliche Salze.

Bevorzugt sind (-)-Enantiomer der Verbindungen der Formel I wobei Ar für

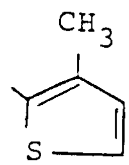

$R^1$ für Wasserstoff,

$R^3$ für Wasserstoff,

$R^4$ für Methyl steht.

Im folgenden wird die vorgenannte Verbindung als Verbindung II bezeichnet.

Geeignete pharmazeutisch verträgliche Salze sind beispielsweise Sulfate, Nitrate, Phosphate, Borate, Hydrochloride und Hydrobromide sowie Salze organischer Sauren wie Azetat, Tartrat, Maleat, Zitrat, Succinat, Benzoat, Ascorbat, Methansulfonat oder alpha-Ketoglutarat. Bevorzugt sind dabei Salze wie Hydrochlorid, Tartrat und Methansulfonat.

Unter dem Begriff Alkanoyl werden Reste der entsprechenden Alkansäuren verstanden, bei denen die Bindung über die entfernte Hydroxy-Gruppe der Carboxylsäure erfolgt. Mit Heteroaryl werden Reste aus der Gruppe der obengenannten Ar-Verbindungen bezeichnet.

Die razemischen Verbindungen der Formel I können wie in der Europäischen Offenlegungsschrift 322 582 beschrieben, hergestellt werden. Verbindungen der Formel I worin $R^1$ Wasserstoff, $R^4$ Wasserstoff und $R^3$ eine Alkyl- oder Phenylgruppe ist, können beispielsweise hergestellt werden, indem man N-(2-methylphenyl)-2,2-dimethylpropanamid über das entsprechende Dilithiumaddukt, mit einem Heteroarylcarboxyaldehydimin zu einem heteroarylsubstituierten Intermediat umsetzt und dann nach Hydrolyse das entsprechende Diamin erhält. N-(2-methylphenyl)-2,2-dimethylpropanamid kann durch die Reaktion von o-Toluidin mit Trimethylacetylchlorid erhalten werden (US 4,374,067). Die Reaktion von primären aliphatischen oder aromatischen Aminen mit Heteroarylcarboxyaldehyden um die entsprechenden Imine zu erhalten, ist bekannt (G. Hilgetag, Preparative Organic Chemistry, John Wiley & Sons, Inc. New York 1972, Seiten 504-509).

Die Enantiomere können aus den razemischen Verbindungen beispielsweise durch Chromatographie an optisch aktivem Säulenmaterial isoliert werden. Hierbei kommen die bekannten Cyclodextrine, polymere Chiralitätsträger wie Chira Spher^R(Firma E. Merck, Darmstadt) und Cellulosederivate in Frage. Prinzipiell kommt auch die Trennung von diastereomeren Salzen in Frage. Dafür eignen sich die optisch aktiv vorliegenden Carbonsäuren wie beispielsweise (+)-Campher-10-sulfonsäure, substituierte (D)- oder (L)-Weinsäurederivate, Mandelsäurederivate, Milchsäurederivate, sowie weitere bekannte optisch aktive Carbonsäuren.

Zur Herstellung einer chiralen Form eignet sich prinzipiell auch das Verfahren, wobei man eine Verbindung der Formel III,

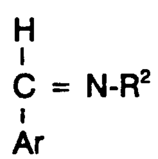

(III)

worin Ar, R oder $R^2$ die oben genannte Bedeutung haben, mit einer Verbindung der Formel IV,

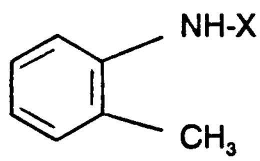

(IV)

worin X eine chirale Schutzgruppe bedeutet, umsetzt oder wenn X keine Chiralität enthält die Verbindung der Formel III mit der Verbindung der Formel IV in Gegenwart eines chiralen Komplexliganden zur Reaktion bringt.

Für analytische Zwecke können die Verbindungen der Formel I, nach bekannten Derivatisierungen, auch durch gaschromatographischen Methoden an chiralen Phasen durch Trennungen der Razemate isoliert werden.

Es ist nicht nötig das (+)-Enantiomere völlig abzutrennen, um die bessere Schutzwirkung des (-)-Enantiomeren zu erzielen. Mischungen der Isomere die mindestens 80 % des (-)-Enantiomeren enthalten können auch schon verwendet werden; insbesondere wenn sie mehr als 95 % des (-)-Enantiomeren enthalten. Besonders bevorzugt sind Präparate die mehr als 99 % des (-)-Enantiomeren enthalten.

Erfindungsgegenstand sind ferner Arzneimittel, die durch eine wirksame Menge des (-)-Enantiomers der Verbindung der Formel I und/oder deren physiologisch verträglichen Salze, gekennzeichnet sind. Die erfindungsgemäßen Verbindungen der Formel I können entweder für sich allein, beispielsweise in Mikrokapseln, in Mischungen untereinander oder vorzugsweise in Kombination mit geeigneten pharmazeutischen Trägerstoffen, Verdünnungsmitteln und/oder anderen Hilfstoffen eingesetzt werden. Die erfindungsgemäßen Arzneimittel können parenteral, intravenös, rektal oder oral verabreicht werden.

Geeignete feste oder flüssige galenische Zubereitungsformen sind beispielsweise Granulate, Pulver, Dragees, Tabletten, (Mikro)kapseln, Suppositorien, Sirupe, Säfte, Suspensionen, Emulsionen, Tropfen oder injizierbare Lösungen sowie Präparate mit protahierter Wirkstoffe-Freigabe, bei deren Herstellung üblicherweise Hilfsmittel, wie Trägerstoffe, Spreng-, Binde-, Überzugs-, Quellungs-, Gleit-, oder Schmiermittel, Geschmackstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, wie Lebertran, Sonnenblumen-, Erdnuß-, oder Sesamöl, Polyethylenglykole und Lösungsmittel, wie etwa steriles Wasser, physiologische Kochsalzlösung und ein- oder mehrwertige Alkohole, z.B. Glycerin genannt.

Vorzugsweise werden die pharmazeutischen Präparate in Dosierungseinheiten hergestellt und verabreicht, wobei jede Einheit als aktiven Bestandteil eine bestimmte Dosis mindestens einer Verbindung gemäß Formel I, gegebenenfalls in Salzform, enthält. Bei festen Dosierungseinheiten, wie Tabletten, Kapseln, Dragees, oder Suppositorien, kann diese Dosis bis zu etwa 300 mg, bevorzugt jedoch etwa 20 bis 100 mg, und bei Injektionslösungen in Ampullenform bis zu etwa 100 mg, vorzugsweise aber etwa 3 bis 30 mg, betragen. Für die Behandlung eines Erwachsenen, etwa 70 kg schweren Patienten sind -je nach Wirksamkeit der Verbindungen gemäß Formel I, bei Mensch und Tier Tagesdosen von etwa 20 bis 1000 mg Wirkstoff, vorzugsweise etwa 50 bis 300 mg, bei oraler Verabreichung und von etwa 20 bis 300 mg, bevorzugt etwa 3 bis 10 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Verbindungen der Formel I, gegebenenfalls ihre physiologisch verträglichen Salze bei der Herstellung der vorgenannten galenischen Zubereitungsformen auch zusammen mit anderen geeigneten Wirkstoffen, beispielsweise durchblutungsfördernden Substanzen, Plättchenaggregationshemmern, Thrombozytenaggregationshemmern und Kalziumantagonisten kombiniert werden.

Die erfindungsgemäßen Arzneimittel eignen sich insbesondere zur Prophylaxe und für die Behandlung von neurodegenerativer Erkrankungen, beispielsweise von Krankheitsformen als Kosequenz von Erkrankungen wie Hirninfarkt mit nur teilweise reversiblen Symptomen (PRIND), ischämischen Zuständen nach Herzstillstand oder chirurgischen Eingriffen, anoxischen Zuständen, Intoxikationen oder Rückenmarksverletzungen, perinataler Asphyxie, altersbedingter neurodegenerativer Erkrankungen, Alzheimer Demenz(SDAT), Schizophrenie, Epilepsie, Huntington-Chorea Amylotrophische Lateralsklerose, Parkinsonsche Erkrankung, sowie anderer neurodegenerativer Erkrankungen.

Überraschend wurde nun gefunden, daß das (-)Enantiomere eine größere Schutzwirkung hat als das (+)Enantiomere oder das Razemat und wegen der geringeren Beeinträchtigung der Zentralen Nervensystem (ZNS)-Funktionen höher dosiert werden kann. Diese unerwartete Eigenschaft wurde durch die

Bestimmung der ZNS-Verträglichkeit und der Bestimmung des Verhaltensscores nach bilateraler Okklusion der Arteria Carotis am Mongolischen Gerbil belegt. Die gute ZNS-Verträglichkeit des (-)-Enantiomers erlaubt seinen therapeutischen Einsatz, im Gegensatz zum (+)-Enantiomer oder dem razemischen Gemisch der Verbindung der Formel I.

Die Erfindung wird nun durch die folgenden Beispiele näher erläutert.

Beispiel 1

N-[2-[2-(Methylamino)-2-(3-methyl-2-thienyl)ethyl]phenyl]-2,2-dimethylpropanamid

In eine gekühlte Lösung (-6°C) von 76 g 2,2-Dimethyl-N-[(2-methyl)phenyl]propanamid in 600 ml von Tetrahydrofuran wurden tropfenweise über 1,25 Stunden 344 ml einer 2,5 M Lösung von n-Butyllithium in Hexan zugefügt. Die erhaltene Lösung wurde unter Kühlung 2 Stunden gerührt und danach wurden tropfenweise über 20 Minuten eine Lösung von 6 g 3-Methyl-2-thiophencarboxyaldehydmethylimin in 30 ml Toluol zugesetzt. Die erhaltene Lösung wurde für weitere 20 Minuten gerührt und durch die schnelle Zugabe von Wasser abgestoppt. Die organischen Lösungsmittel wurden abdestilliert und das verbleibende Öl-Wassergemisch wurde mit Dichlormethan extrahiert. Die vereinigten organischen Phasen wurden über wasserfreiem Magnesiumsulfat getrocknet, filtriert und zu einem Öl eingeengt.

Die Reinigung erfolgte über präparative HPLC (Water's Associates Prep LC/System 500, Silicagel, Laufmittel Ethylacetat).
Es wurden 70 g von N-[2-[2-(Methylamino)-2-(3-methyl-2-thienyl)ethyl]phenyl]-2,2-dimethylpropanamid als Öl erhalten.

| Analyse | | | | |
|---|---|---|---|---|
| $C_{19}H_{26}N_2O_3$ | Berechnet<br>Gefunden | 69,05 % C;<br>68,80 % C; | 7,93 % H;<br>7,80 % H; | 8,48 % N<br>8,13 % N |

Beispiel 2

2-Amino-N-methyl-α-(3-methyl-2-thienyl)benzylethanamin (Verbindung II)

Eine Lösung von 35 g von der erhaltenen Verbindung aus Beispiel 1 wurde in 600 ml einer 6 N HCl für 8 Stunden zum Sieden erhitzt. Die Lösung wurde danach in ein Eis-Wasser-Gemisch dekantiert und mit einer 50 % NaOH-Lösung alkalisch gestellt. Die wäßrige Lösung wurde mit Dichlormethan (2 L) extrahiert, über wasserfreiem Magnesiumsulfat getrocknet und zu einem Öl eingeengt. Die Reinigung erfolgte über preparative HPLC (Water's Associates Prep LC/System 500 A, Silicagel, Laufmittel Methanol). Es wurden 6,42 g von 2-Amino-N-methyl-α-(3-methyl-2-thienyl)benzylethanamin als Öl erhalten.

| Analyse | | | | |
|---|---|---|---|---|
| $C_{14}H_{18}N_2S$ | Berechnet<br>Gefunden | 68,25 % C;<br>68,35 % C; | 7,36 % H;<br>7,26 % H; | 11,37 % N<br>11,35 % N |

Beispiel 3

Chromatographische Trennung des Razemats:

1 g der razemischen Verbindung II wird an einer Säule (3,6 x 90 cm), die mit Triacetylcellulose (E. Merck, Darmstadt) gefüllt ist mit Ethanol bei einer Fließgeschwindigkeit von 2 ml/min. getrennt. Dabei wird zunächst das (-)Enantiomere eluiert und nachfolgend das (+)Enantiomere. Beide Enantiomere werden anschließend mit ethanolischer HCl in ihr Dihydrochlorid überführt und aus Ethanol umkristallisiert. Man erhält jeweils 400 mg des Enantiomers mit einen $alpha_{30}D$-Wert in Methanol von 35,936°. Der Schmelzpunkt der Dihydrochloride beider Enantiomere liegt bei 214°C. Das $^1$HNMR- und IR Spektrum zeigen die zu erwartenden Signale bzw. Banden.

| Analyse | | | | | |
|---|---|---|---|---|---|
| $C_{14}H_{20}Cl_2N_2S$ | Berechnet<br>Gefunden | 52,66 % C;<br>52,50 % C; | 6,31 % H;<br>6,42 % H; | 8,77 % N;<br>8,49 % N; | 22,21 % Cl<br>22,46 % Cl |

Biologische Wirksamkeit:

1) Neuroprotektion nach Vorderhirn-Ischämie am Gerbil

Anhand der nachfolgend beschriebenen Versuchsdurchführungen (K. Rudolphi et. al, J. Cereb. Blood Flow Metab., 7:74. 1987) ergibt sich, daß die unten aufgeführten Verbindungen in der Lage sind, zerebrale Nervenzellen gegen eine schwere ischämische Schädigung zu schützen.

Bei 20 männlichen Mongolischen Gerbils werden unter Halothan-Narkose durch beidseitigen Verschluß der A. Carotis mittels Aneurysmaclips eine 5-minütige Ischämie des Vorderhirns mit anschließender Rezirkulation erzeugt. Die Tiere werden 45 Minuten lang nach der Ischämie beobachtet und das Ausmaß der neurologischen Symptome mittels eines 5-stufigen Verhaltensscores bestimmt. 7 Tage später werden die Tiere unter Halothan-Narkose dekapitiert, die Gehirne in Carnoy-Lösung immersionsfixiert und das Ausmaß des Neuronenschadens in der $CA_1$-Region des Hippocampus mit Hilfe eines 5-stufigen histopathologischen Scores "blind" bestimmt. Die Prüfsubstanzen werden je einer Gruppe von 10 Gerbils intraperitoneal oder per os 15 Minuten vor der Ischämie verabreicht. Pro Testsubstanz wird eine ischämische Kontrollgruppe von ebenfalls 10 Tieren mit dem jeweiligen Lösungsmittel behandelt. Die Ergebnisse sind in Tabelle 6 als % Änderung des mittleren neurologsichen Verhaltensscores bzw. histopathologischen Scores (Summe der individuellen Scores/Anzahl der Tiere) der behandelten vs. Kontrollgruppe zusammengefaßt.

Tabelle 1

| Verhaltensscore nach fünfminütiger Vorderhirn-Ischämie am Mongolischen Gerbil | | | |
|---|---|---|---|
| Verbindung | Dosis (mg/kg) | Verhaltensscore (% Änderung) | Zellnekrosen (% Änderung) |
| Razemisch II | 30 ip. | -15 | -75 |
| (+)II | 30 ip. | -51 | -90 |
| (-)II | 30 ip. | -32 | - 9 |
| (-)II | 60 ip. | -75 | -23 |

Je höher der Verhaltensscore (-% Änderungen), desto besser ist die Erholung der Tiere nach Ischämie.

2) ZNS-Verträglichkeit am gesunden Tier (Maus)

Adulten männlichen NMRI-Mäusen werden die zu untersuchenden Substanzen in Dosen von 0,1-100 mg/kg i.p. in einem Volumen von 10 ml/kg verabreicht. Pro Versuchsdosis werden drei Tiere eingesetzt, wobei eine Gruppe als Placebo nur mit dem Lösungsmittel behandelt (Kontrolle) mitgeführt wird.

Die Placebogruppe dient dabei zur zeitgleichen Verhaltensfeststellung, parallel zur Versuchsgruppe. Sofort nach der Applikation werden die Tiere in Dreiergruppen in Beobachtungskäfige gesetzt und drei Stunden lang am ersten Tag beobachtet. Anschließend werden die Tiere gefüttert, getränkt und weitere sechs Tage, jeweils nach 24 Stunden, für eine halbe Stunde beobachtet. Dabei ergaben sich folgende Befunde:

Tabelle 2

| Beobachtete Phänomene (3 Stunden nach Applikation) | | |
|---|---|---|
| Verbindung | Dosis mg/kg i.p. | Symptome |
| Razemisch II | 0,1-3<br>1,0-3,0<br>10-100 | vermehrter Putztrieb<br>Unruhe, Erregung, vermehrter Putztrieb<br>Taumeln, vermehrter Putztrieb, Unruhe, Erregung |
| (+)II | 0,1-0,3<br>1,0-3,0<br>10-100 | o. B.<br>kurzandauernde Erregung<br>vermehrter Putztrieb, Unruhe, Erregung, Taumeln |
| (-)II | 0,01-0,3<br>1,0-3,0<br>10-100 | o. B.<br>o. B.<br>Unruhe, vermehrter Putztrieb |
| o.B. steht für ohne Befund, i.p. für intraperitoneal, i.v. für intravenös. | | |

Das Racemat und die (+)-Form zeigen ab 10 mg/kg i. p. eine ZNS erregende Aktivität, die als unverträglich gilt, während die (-)-Form ein vergleichbares das normale Verhalten des Tieres verändernde Wirkung, erst ab 100 mg/kg i. p. aufweist.

**Patentansprüche**

**1.** (-)-Enantiomer der Verbindung der Formel I,

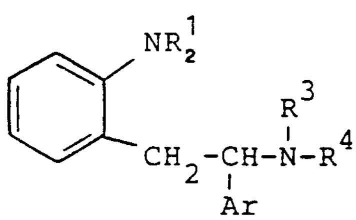

wobei Ar für Verbindungen aus der Gruppe

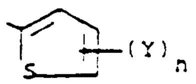, 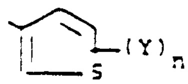, 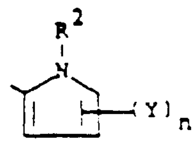, 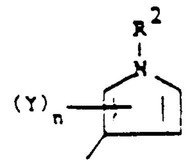

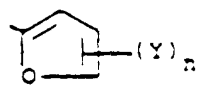, 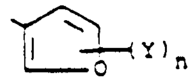,

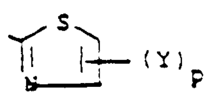, 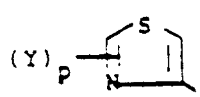, 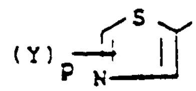,

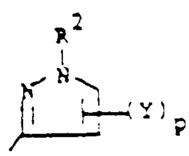, 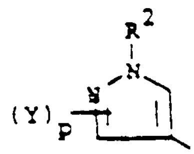, 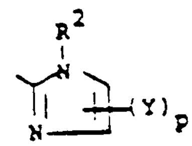,

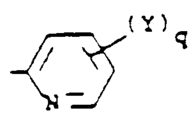, 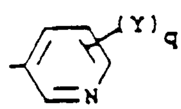, 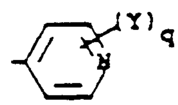

worin Y für
1) Halogen, wie Fluor, Chlor, Brom oder Jod,
2) Hydroxy,
3) $(C_1-C_5)$-Alkyl, geradkettig oder verzweigt,
4) $(C_1-C_5)$-Alkoxy, geradkettig oder verzweigt,
5) Trifluormethyl,
n für eine ganze Zahl von 0 bis 3,
p für eine ganze Zahl von 0 oder 1,
q für eine ganze Zahl von 0 bis 4,
$R^2$ für
1) Wasserstoff,
2) $(C_1-C_5)$-Alkyl, geradkettig oder verzweigt,
3) $(C_1-C_5)$-Alkanoyl, geradkettig oder verzweigt,
$R^1$ für
1) Wasserstoff,
2) $(C_1-C_5)$-Alkyl,
$R^3$ für
1) Wasserstoff,
2) $(C_1-C_5)$-Alkyl,
3) Phenyl,
4) Phenyl, ein- oder mehrfach substituiert durch
4.1 $(C_1-C_4)$-Alkyl
4.2 Halogen, wie Fluor, Chlor oder Brom,
4.3 Methoxy,
4.4 Hydroxy,
4.5 Trifluormethyl,
$R^4$ für
1) Wasserstoff,
2) $(C_1-C_5)$-Alkyl,

3) -C(O)-$R^5$,
$R^5$ für
1) Wasserstoff,
2) ($C_1$-$C_5$)-Alkyl,
3) Allyl,
steht und/oder deren pharmazeutisch verträgliche Salze.

2. (-)-Enantiomer der Verbindung der Formel I nach Anspruch 1, dadurch gekennzeichnet, daß Ar für

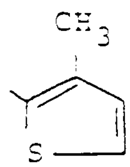

$R^1$ für Wasserstoff,
$R^3$ für Wasserstoff und
$R^4$ für Methyl steht.

3. Eine Mischung der (-)- und (+)-Enantiomere der Verbindung der Formel I nach Anspruch 1 oder 2, dadurch gekennzeichnet, daß mindestens 99 % der (-)-Enantiomeren vorhanden sind.

4. Verfahren zur Herstellung der (-)-Enantiomere der Verbindung der Formel I nach Anspruch 1 oder 2 oder der Mischung nach Anspruch 3, dadurch gekennzeichnet, daß das Razemat der Verbindung der Formel I durch Chromatographie an optisch aktivem Säulenmaterial in die (+)- und (-)-Enantiomere aufgetrennt wird.

5. Arzneimittel, gekennzeichnet durch einen wirksamen Gehalt an einem oder mehreren (-)-Enantiomer der Verbindung der Formel I nach Anspruch 1 oder 2 oder der Mischung nach Anspruch 3.

6. Arzneimittel nach Anspruch 5, dadurch gekennzeichnet, daß sie zur Prophylaxe und/oder Therapie von neurodegenerativen Erkrankungen eingesetzt werden.

7. Verwendung von dem (-)-Enantiomer der Verbindung der Formel I nach Anspruch 1 oder 2 oder der Mischung nach Anspruch 3 zur Herstellung eines Arzneimittels zur Prophylaxe und/oder Therapie von neurodegenerativen Erkrankungen.

8. Verfahren zur Herstellung eines Arzneimittels nach Anspruch 5 oder 6, dadurch gekennzeichnet, daß man mindestens ein (-)-Enantiomer der Verbindung der Formel I mit einem physiologisch annehmbaren Träger, sowie gegebenenfalls geeigneten Zusatz- und/oder Hilfsstoffen in eine geeignete Darreichungsform bringt.

**Patentansprüche für folgende Vertragsstaaten : ES, GR**

1. Verfahren zur Herstellung des (-)-Enantiomers der Verbindung der Formel I,

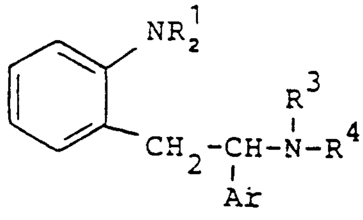

wobei Ar für Verbindungen aus der Gruppe

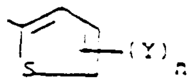 , 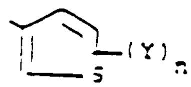 , 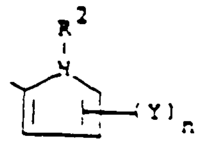 , 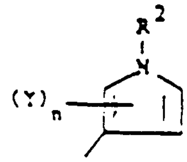

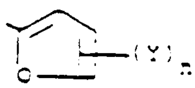 , 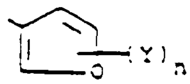 ,

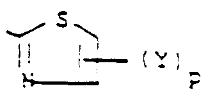 , 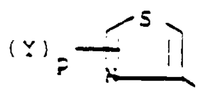 , 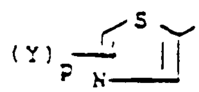 ,

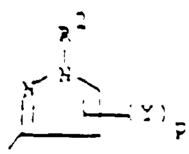 , 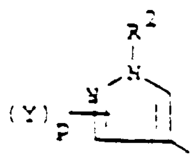 , 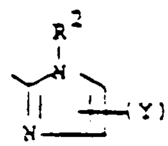 ,

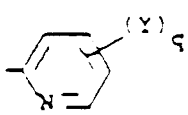 , 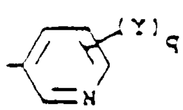 , 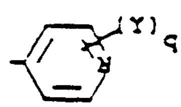

worin Y für
- 1) Halogen, wie Fluor, Chlor, Brom oder Jod,
- 2) Hydroxy,
- 3) $(C_1-C_5)$-Alkyl, geradkettig oder verzweigt,
- 4) $(C_1-C_5)$-Alkoxy, geradkettig oder verzweigt,
- 5) Trifluormethyl,

n für eine ganze Zahl von 0 bis 3,
p für eine ganze Zahl von 0 oder 1,
q für eine ganze Zahl von 0 bis 4,
$R^2$ für
- 1) Wasserstoff,
- 2) $(C_1-C_5)$-Alkyl, geradkettig oder verzweigt,
- 3) $(C_1-C_5)$-Alkanoyl, geradkettig oder verzweigt,

$R^1$ für
- 1) Wasserstoff,
- 2) $(C_1-C_5)$-Alkyl,

$R^3$ für
- 1) Wasserstoff,
- 2) $(C_1-C_5)$-Alkyl,
- 3) Phenyl,
- 4) Phenyl, ein- oder mehrfach substituiert durch
  - 4.1 $(C_1-C_4)$-Alkyl
  - 4.2 Halogen, wie Fluor, Chlor oder Brom,
  - 4.3 Methoxy,
  - 4.4 Hydroxy,
  - 4.5 Trifluormethyl,

$R^4$ für
1) Wasserstoff,
2) ($C_1$-$C_5$)-Alkyl,
3) -C(O)-$R^5$,
$R^5$ für
1) Wasserstoff,
2) ($C_1$-$C_5$)-Alkyl,
3) Allyl,
steht und/oder deren pharmazeutisch verträgliche Salze, dadurch gekennzeichnet, daß das Razemat der Verbindung der Formel I durch Chromatographie an optisch aktivem Säulenmaterial in die (+)- und (-)-Enantiomere aufgetrennt wird.

**2.** Verfahren zur Herstellung des (-)-Enantiomers der Verbindung der Formel I nach Anspruch 1, dadurch gekennzeichnet, daß Ar für

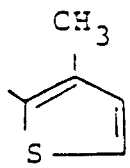

$R^1$ für Wasserstoff,
$R^3$ für Wasserstoff und
$R^4$ für Methyl steht.

**3.** Verfahren zur Herstellung einer Mischung der (-)- und (+)-Enantionmere der Verbindung der Formel I nach Anspruch 1 oder 2, dadurch gekennzeichnet, daß mindestens 99 % der (-)-Enantiomeren vorhanden sind.

**4.** Verfahren zur Herstellung eines Arzneimittels, gekennzeichnet durch einen wirksamen Gehalt an einem oder mehreren (-)-Enantiomer der Verbindung der Formel I erhalten nach Anspruch 1 oder 2 oder der Mischung nach Anspruch 3, dadurch gekennzeichnet, daß man mindestens ein (-)-Enantiomer der Verbindung der Formel I mit einem physiologisch annehmbaren Träger, sowie gegebenenfalls geeigneten Zusatz- und/oder Hilfsstoffen in eine geeignete Darreichungsform bringt.

**5.** Verfahren nach Anspruch 4, dadurch gekennzeichnet, daß die erhaltene Verbindung zur Prophylaxe und/oder Therapie von neurodegenerativen Erkrankungen eingesetzt wird.

**6.** Verwendung von dem (-)-Enantiomer der Verbindung der Formel I nach Anspruch 1 oder 2 oder der Mischung nach Anspruch 3 zur Herstellung eines Arzneimittels zur Prophylaxe und/oder Therapie von neurodegenerativen Erkrankungen.

Europäisches Patentamt

# EUROPÄISCHER RECHERCHENBERICHT

Nummer der Anmeldung

EP 92 10 7394

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | CHEMICAL ABSTRACTS, vol. 114, no. 9, 4. März 1991, Columbus, Ohio, US; abstract no. 75041V, S. FIELDING ET AL.: 'Preclinical anticonvulsant and neuroprotective profile of 8319, a noncompetetive NMDA antagonist' Seite 54 ; Spalte 2 ; * Zusammenfassung * & PROG. CLIN. BIOL. RES. Bd. 361, 1990, Seiten 533 - 541; | 1,2,6 | C07D333/20<br>C07B57/00<br>A61K31/38 |
| D,Y | EP-A-0 322 582 (HOECHST-ROUSSEL PHARMACEUTICALS INCORPORATED) * Seite 4, Zeile 57 - Seite 7, Zeile 11; Ansprüche 8-14; Beispiele 14,15 * | 1,2,6 | |
| P,Y | WO-A-9 205 169 (FISONS CORPORATION) * Seite 1, Zeile 1 - Seite 3, Zeile 27 * * Seite 4, Zeile 1 - Zeile 26 * * Zusammenfassung; Anspruch 1 * | 1,2,6 | |
| | | | **RECHERCHIERTE SACHGEBIETE (Int. Cl.5 )** |
| | | | C07D<br>C07B |

Der vorliegende Recherchenbericht wurde für alle Patentansprüche erstellt

| Recherchenort | Abschlußdatum der Recherche | Prüfer |
|---|---|---|
| DEN HAAG | 31 JULI 1992 | B. Paisdor |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)